# EUROPEAN PATENT APPLICATION

(11) **EP 2 295 967 A1**
(43) Date of publication of application: **16.03.2011**
(21) Application number: 09758266.2
(22) Date of filing: 29.05.2009
(51) Int. Cl.: G01N 33/53

(54) **IgA NEPHROPATHY DETECTION METHOD AND DETECTION KIT**

(30) Priority: 02.06.2008 JP 2008144883
(71) Applicant: Eisai R&D Management Co., Ltd., Tokyo 112-8088 (JP)
(72) Inventor: OBARA, Takashi, Tsukuba-shi Ibaraki 300-2635 (JP); MIZOGUCHI, Sadaaki, Tsukuba-shi Ibaraki 300-2635 (JP); HOTTA, Osamu, Sendai-shi Miyagi 983-0823 (JP)
(74) Representative: Woods, Geoffrey Corlett
(86) International application number: PCT/JP2009/059873
(87) International publication number: WO 2009/147999

(57) **Abstract**

There is provided a renal disease testing method comprising a complex detection step of detecting a complex of an antigen derived from human renal mesangial cells and human IgA in a sample derived from urine collected from a subject. It is preferred that the renal disease testing method of the invention further comprises a determination step of assessing the whether the renal disease is IgA nephropathy based on the ratio of the amount of the complex detected in the complex detection step to the amount of urinary proteins in the sample. The renal disease testing method of the invention has good detection sensitivity and specificity, and can conveniently and safely assess the existence of a renal disease (preferably, IgA nephropathy).

## Description

### TECHNICAL FIELD

The present invention relates to a method of detecting IgA nephropathy and a test kit.

### BACKGROUND ART

IgA nephropathy is a disease that was reported by Berger, et al., in 1968, and is the most frequent kidney disease among chronic glomerulonephritis other than diabetic nephropathy (for example, see Non-Patent Document No. 1). IgA nephropathy is a disease with a poor long-term prognosis and it is estimated that about 25% of patients with terminal renal failure who are in need of dialysis treatment have IgA nephropathy as the original disease. Although there are methods for delaying the progress of IgA nephropathy, no therapeutic method of treating the disease has been found. Recently, however, a treatment method has been established by Hotta, et al., by which complete remission is achieved if the treatment is started at an early stage.

Meanwhile, according to the "Joint Committee of the Specified Progressive Diseases Investigative Research Division for Progressive Renal Diseases of the Ministry of Health and Welfare and the Japanese Society of Nephrology", while an analysis of urine (continuous microscopic hematuria, continuous or intermittent proteinuria, macroscopic hematuria) and a blood test (blood serum IgA value is 350 mg/dL or more) are employed as supplementary diagnostic criteria, confirmation of IgA precipitation in a glomerular mesangial area based on kidney biopsy is the only means for definite diagnosis of IgA nephropathy. However, as kidney biopsy requires hospitalization for a week or so and is a risky method accompanied by severe bleeding, the diagnosis rate is not necessarily high. Thus, a detection method by which early IgA nephropathy can be conveniently and safely diagnosed is needed.
Further, although methods such as those using an IgA-fibronectin complex are known as diagnosis methods for IgA nephropathy (for example, see Japanese Patent Application Laid-Open (JP-A) No. 2000-241431, Japanese Patent (JP-B) No. 2592121, and Cederholm et al., Proc. Natl. Acad. Sci., 85: 4865-8, 1988), until now no practical method has been developed.

### DISCLOSURE OF THE INVENTION

### PROBLEMS TO BE SOLVED BY THE INVENTION

An object of the invention is to provide an IgA nephropathy testing method having good detection sensitivity and specificity which can assess conveniently and safely the existence of IgA nephropathy and a test kit.

### MEANS FOR SOLVING THE PROBLEMS

As a result of producing an antibody which is specific to an antigen derived from human renal mesangial cells and measuring the quantity of a complex of the antigen derived from human renal mesangial cells and IgA in human urine by using the antibody, the inventors of the invention found that the complex was present in higher concentration in an IgA nephropathy patient compared to a healthy person or a patient having a renal disease other than IgA nephropathy, and therefore completed the invention.

Thus, the specific means for solving the problems above is shown below.
According to a first aspect of the invention, there is provided an IgA nephropathy testing method comprising a complex detection step of detecting a complex of an antigen derived from human renal mesangial cells and human IgA in a sample derived from urine collected from a subject. It is preferable that the complex detection step comprise bringing the sample into contact with an antibody against the antigen derived from human renal mesangial cells and an antibody against human IgA. Furthermore, it is preferable that the method further comprise a step of obtaining the ratio of the amount of the complex detected in the complex detection step to the amount of urinary proteins in the sample derived from urine collected from the subject. It is more preferable that the method further comprise a determination step of assessing an existence of IgA nephropathy based on the ratio above.

According to a second aspect of the invention, there is provided a renal disease testing method comprising a complex detection step of detecting a complex of an antigen derived from human renal mesangial cells and human IgA in a sample derived from urine collected from a subject. It is preferred that the complex determination step comprises brining the sample into contact with an antibody against the antigen derived from human renal mesangial cells and an antibody against human IgA. It is more preferable that the method further comprise a determination step of assessing an existence of a renal disease based on the detected amount of the complex.

According to a third aspect of the invention, there is provided an IgA nephropathy testing method comprising a complex detection step of detecting a complex of an antigen derived from human renal mesangial cells and human IgA in a sample derived from urine collected from a subject, a first determination step of assessing the existence of a renal disease based on the amount of the complex detected in the complex detection step or the amount of urinary proteins in the sample, and a second determination step of assessing whether the renal disease is IgA nephropathy based on the ratio of the amount of the complex detected in the complex detection step to the amount of urinary proteins in the sample.
The complex detection step in the testing method of the invention is preferably an immunochemical method using an antibody against the antigen derived from human renal mesangial cells and an antibody against human IgA, and more preferably a sandwich method using the same.

According to a fourth aspect of the invention, there is provided a method of detecting a complex of an antigen derived from human renal mesangial cells and human IgA, which comprises a step of bringing the sample derived from urine collected from a subject into contact with an antibody against the antigen derived from human renal mesangial cells and an antibody against human IgA. It is preferable that the complex is detected by an immunochemical method using an antibody against the antigen derived from human renal mesangial cells and an antibody against human IgA. It is more preferable that the complex is detected by a sandwich method using the same.
Further, the subject is preferably a person who has developed a renal disease, a person who is suspected to have developed a renal disease, or a person who has a possibility of developing a renal disease. The renal disease is preferably IgA nephropathy.

According to a fifth aspect of the invention, there is provided an test kit for a renal disease that comprises at least an antibody against an antigen derived from human renal mesangial cells and an antibody against human IgA. The renal disease is preferably IgA nephropathy.

### EFFECTS OF THE INVENTION

According to the invention, an IgA nephropathy testing method having good detection sensitivity and specificity which can conveniently and safely assess the existence of IgA nephropathy, and a test kit are provided.

### BRIEF DESCRIPTION OF THE DRAWINGS

Fig. 1 is a distribution chart showing the measurement values of IgA-6C4 recognizing antigen complex in urine which is detected by ELISA.
Fig. 2 is a diagram showing the results obtained from ROC analysis of the measurement values of IgA-6C4 recognizing antigen complex in urine which is detected by ELISA.
Fig. 3 is a distribution chart showing the measurement values of IgA-6C4 recognizing antigen complex in urine which is detected by ELISA, the measurement values being calibrated against the concentration of urinary protein.
Fig. 4 is a diagram showing the results obtained from ROC analysis of the measurement values of IgA-6C4 recognizing antigen complex in urine which is detected by ELISA, in which the measurement values are calibrated against the concentration of urinary protein.
Fig. 5 is a distribution chart showing the measurement values of IgA-4H3 recognizing antigen complex in urine which is detected by ELISA.
Fig. 6 is a diagram showing the results obtained from ROC analysis of the measurement values of IgA-4H3 recognizing antigen complex in urine which is detected by ELISA.
Fig. 7 is a distribution chart showing the measurement values of IgA-4H3 recognizing antigen complex in urine which is detected by ELISA, in which the measurement values are calibrated against the concentration of urinary protein.
Fig. 8 is a diagram showing the results obtained from ROC analysis of the measurement values of IgA-4H3 recognizing antigen complex in urine which is detected by ELISA, in which the measurement values are calibrated against the concentration of urinary protein.

### BEST MODES FOR CARRYING OUT THE INVENTION

In the following, the exemplary embodiments of the invention will be specifically described. However, the scope of the invention is not limited thereto.
The renal disease testing method of the invention comprises a complex detection step of detecting a complex of an antigen derived from human renal mesangial cells and human IgA, from a sample derived from urine collected from a subject. By detecting the complex from the sample derived from urine, a renal disease of the subject may be detected with good detection sensitivity and good specificity. In this context, the renal disease includes IgA nephropathy and renal diseases other than IgA nephropathy.

According to the invention, the sample may be urine itself which is collected from a subject or urine obtained after a treatment like dilution, concentration, and the like which is usually performed on collected urine. According to the invention, the preferred sample is a urine sample obtained by diluting urine collected from a human based on a general method.
Furthermore, the sample derived from urine collected from the subject may be obtained by brining the urine sample above into contact with an antibody against an antigen derived from human renal mesangial cells or into contact with an antibody against human IgA.

Furthermore, the antigen derived from human renal mesangial cells described in the invention is an antigen present in cultured human renal mesangial cells, mesangial cells isolated from a human kidney, or a cell extract which is extracted from them, and it is not specifically limited if it can form a complex with IgA and is an antigen present in urine collected from a subject having a renal disease. Among these, from the viewpoint of specificity of detecting a renal disease and IgA nephropathy, it is preferably an antigen that is distinguished from antigens derived from glomerular-constituting cells other than human renal mesangial cells. It is more preferably an antigen that is distinguished from antigens derived from epithelial cells of the human proximal renal tubule.

Furthermore, the cell extract is not specifically limited if it is extracted from cultured human renal mesangial cells or mesangial cells isolated from a human kidney. From the viewpoint of specificity of detecting a renal disease and IgA nephropathy, it is preferably an extract of cytoskeletal components.
The cell extract may be prepared according to a method generally known in the art. Specific examples include a method of using ProteoExtract Subcellular Proteome Extraction Kit (trade name, manufactured by Calbiochem), or the like.

The complex detecting method is not specifically limited, and a protein detecting method generally used in the art may be used. Preferably, it is a method which allows quantification or semi-quantification of a complex to be detected.

According to the invention, the apparatus which is used for the complex detection is not specifically limited, and it may be appropriately selected depending on a complex testing method. Specific examples include an HPLC apparatus, an apparatus for mass spectrometry, an apparatus for electrophoresis (an apparatus for capillary electrophoresis, and the like), a full-automatic or semi-automatic apparatus for enzyme immunoassay, a cell washer, a full-automatic or semi-automatic apparatus for chemiluminescence immunoassay, an apparatus for detecting luminescence, a full-automatic or semi-automatic apparatus for electro-chemiluminescence immunoassay, an apparatus for optical measurement, a plate reader, a CCD camera, a full-automatic or semi-automatic apparatus for fluorescence immunoassay, an apparatus for fluorescence measurement, a full-automatic or semi-automatic apparatus for radioimmunoassay, a liquid scintillation counter, a coulter counter, an apparatus for measuring surface plasmons, and an apparatus for blotting and a densitometer.

From the viewpoint of sensitivity, specificity and convenience of detection, in the invention, an immunochemical method using an antibody binding to the complex is preferable. In the specification of the invention, the term "antibody against the complex" may have the same definition as the general meaning of antibody like in "antibody which recognizes the complex" and "antibody which binds to the complex." Specifically, the "antibody against the complex" binds to at least part of the complex consisting of an antigen derived from human renal mesangial cells and human IgA to form a new complex. Herein below, the same holds true for the expressions the "antibody against the antigen derived from human renal mesangial cells" and the "antibody against human IgA."
As for the immunochemical method used in the invention, a method that is generally used in the art may be used without any limitation. Specific examples include enzyme immunoassay (ELISA), chemiluminescence immunoassay, electro-chemiluminescence immunoassay, absorbance determination, fluorescent antibody method, radioimmunoassay (RIA), surface plasmon resonance, Western blot and dot blot. From the viewpoint of sensitivity, specificity and convenience of detection, it is preferable to use enzyme immunoassay (ELISA) in the invention.

As for an immunochemical method used in the invention, from the viewpoint of specificity and convenience, a sandwich method using an antibody that recognizes and binds to an antigen derived from human renal mesangial cells in the complex and an antibody that recognizes and binds to human IgA in the complex is preferable.

The sandwich method may be carried out as follows, for example. An antibody that binds to the complex (i.e., a primary antibody) is immobilized on a carrier like a plate, and the like. Generally, in order to block a non-specific binding site in the plate or the like, a blocking process is performed by using a protein such as casein or a surfactant. Subsequently, a sample derived from urine or a test sample is added and incubated. After washing the plate, as an antibody which binds to the complex, a labeled secondary antibody is added followed by incubation. Then, by washing the plate or the like and detecting the label, the process may be carried out.

From the viewpoint of detection sensitivity and specificity, it is preferable that two kinds of antibodies selected from antibodies that bind to the complex are used as the primary antibody and the secondary antibody described above. It is more preferable that the antibody against an antigen derived from human renal mesangial cells is used as one of the primary antibody and the secondary antibody described above while the antibody against human IgA is used as the other antibody. It is still more preferable that the antibody against an antigen derived from human renal mesangial cells is used as the primary antibody while the antibody against human IgA is used as the secondary antibody.

According to the invention, the antibody that binds to the complex may be either a polyclonal antibody or a monoclonal antibody. From the viewpoint of specificity for detecting a renal disease, a monoclonal antibody is preferable.
The antibody that binds to the complex may be produced according to a method generally performed in the art.

The polyclonal antibody against an antigen derived from human renal mesangial cells may be produced according to a general producing method used in the art. It may be obtained as follows, for example. An animal such as a rabbit or a goat is immunized with cultured human renal mesangial cells, mesangial cells isolated from a human kidney, or extract thereof to obtain blood serum, which is then purified by ammonium sulfate precipitation, Protein A column, Protein G column, DEAE ion exchange chromatography, an affinity column to which an extract of human renal mesangial cells is attached, or the like for the production.
According to the invention, the antibody having higher specificity to an antigen derived from human renal mesangial cells, from which antibodies which bind to antigens other than the antigen derived from human renal mesangial cells are removed by a general method used in the art, for example, an absorption process, and the like is preferable.

Furthermore, for producing the monoclonal antibody against an antigen derived from human renal mesangial cells, a small animal such as a mouse is immunized with cultured human renal mesangial cells, mesangial cells isolated from a human kidney, or an extract thereof, then the spleen is harvested from the mouse and homogenized to isolate the cells, which are then fused with mouse myeloma cells by using a reagent such as polyethylene glycol and from the resulting fused cells (i.e., hybridoma) a clone producing an antibody that binds to an antigen derived from human renal mesangial cells is selected. Subsequently, the selected hybridoma is introduced to an abdominal cavity of a mouse, and the monoclonal antibody that is obtained from the ascites recovered from the same animal is purified by ammonium sulfate precipitation, Protein A column, Protein G column, DEAE ion exchange chromatography, an affinity column to which an extract of human renal mesangial cells is attached, or the like.

With respect to selection of the clone producing the monoclonal antibody that binds to an antigen derived from human renal mesangial cells, it is preferable that a clone exhibiting higher reactivity to the antigen derived from human renal mesangial cells is selected. In addition to this, it is preferable that a clone that produces an antibody that also binds to an antigen other than the antigen derived from human renal mesangial cells is excluded. Examples of the antigen other than the antigen derived from human renal mesangial cells include an antigen derived from cells that have a high possibility of being contaminated in urine and which are not human renal mesangial cells, and a preferable example is an antigen derived from epithelial cells of human proximal renal tubule.

In the present invention, the antibody against an antigen derived from human renal mesangial cells is preferably an antibody that binds to the antigen derived from human renal mesangial cells but not to an antigen derived from cells having a high possibility of being contaminated in urine that are not human renal mesangial cells, from the viewpoint of specificity for detecting a renal disease and IgA nephropathy. An antibody that binds to an antigen derived from human renal mesangial cells but not to an antigen derived from epithelial cells of the human proximal renal tubule is more preferable.
Furthermore, the antibody against an antigen derived from human renal mesangial cells may be any of an antibody derived from a small animal such as a mouse as obtained above, a chimeric antibody, a humanized antibody, a fully human antibody, or the like.

As for the antibody against human IgA, it is not specifically limited so long as it is an antibody capable of binding to IgA in the complex. It may be any one that recognizes the H chain, the J chain, the secretory component, or the like of human IgA.
Furthermore, the antibody against human IgA may be either a polyclonal antibody or a monoclonal antibody. However, from the viewpoint of detection specificity for the complex, a monoclonal antibody against human IgA is preferable.
The monoclonal antibody against human IgA may be produced by using human IgA as an antigen in the same manner as the antibody against the antigen derived from human renal mesangial cells. Furthermore, as for the antibody against human IgA, a commercially available anti-human IgA antibody may be used.
Still furthermore, the antibody against human IgA may be any of an antibody derived from a small animal such as a mouse as obtained in the above, a chimeric antibody, a humanized antibody, a fully human antibody, or the like.

As for the label, any label well known in the art may be used without specific limitation. Examples include an enzyme, a chemiluminescent substance, an electro-chemiluminescent substance, a radioactive substance, and the like.

According to the invention, from the viewpoint of detection sensitivity and convenience, it is preferable to use an enzyme as the label.
The enzyme is not specifically limited so long as it is an enzyme that can be quantified by a physical or a chemical method. Examples include an enzyme such as alkaline phosphatase, horseradish peroxidase (HRP) and luciferase.

Furthermore, a method of detecting the label is not specifically limited so long as it is a method that allows for quantification or semi-quantification of the label, and it may be appropriately selected depending on type of the label. Examples include absorbance, luminescence intensity, fluorescence intensity, and radioactivity count.
Based on the quantification or semi-quantification of the label, quantification or semi-quantification of the complex may be achieved.

When the complex detection step of the invention is carried out with absorbance determination for detecting the label using enzyme immunoassay (ELISA) as a measurement apparatus for the complex detection that is preferably used in the invention, an absorbance determination apparatus that allows the measurement of absorbance of a chromophore generating from the label that is bound to the complex and is equipped with a sample loading part in which a sample containing the chromophore generating from the label is loaded, a light irradiating part in which light is irradiated to the sample, and a light quantity measuring part in which at least one of reflected light and transmitted light is collected from the sample and the collected light quantity is measured can be mentioned.
According to the invention, by irradiating light to the chromophore that is generated from the label bound to the complex of an antigen derived from human renal mesangial cells and human IgA in a test sample (i.e., a sample derived from urine collected from a subject) through the light irradiating part and quantifying the absorbance of the chromophore through the light quantity measuring part, the content of the complex of the antigen derived from human renal mesangial cells and human IgA in the sample may be measured.

Furthermore, the label detection may be also carried out by detecting the light emission from a sample instead of the absorbance. In such case, instead of the apparatus for measuring absorbance described above, an apparatus which allows the measurement of light emitted from the label bound to the complex itself or from the substrate in a reaction liquid is used, examples of which include a measurement apparatus with a sample loading part in which a sample containing the label is loaded and a light quantity measuring part in which emitted light is collected from the sample and the collected light quantity is measured.
According to the invention, by quantifying the light emitted from a sample containing the label that is bound to the complex of an antigen derived from human renal mesangial cells and human IgA contained in a test sample (i.e., a sample derived from urine collected from a subject) through a light quantity measuring part, the content of the complex of the antigen derived from human renal mesangial cells and human IgA contained in a test sample may be measured.

The renal disease testing method of the invention preferably includes a step to assess the existence of a renal disease based on the detected amount of the complex contained in a sample derived from urine. As for the detected amount of the complex, so long as it is the concentration of the complex contained in a sample derived from urine or the quantitative or a semi-quantitative value corresponding thereto, it may be used without specific limitation. Examples include a measurement value obtained by directly measuring the detected amount of the complex, a measurement value obtained by indirectly measuring the detected amount of the complex via detection of the label, and the like.

Furthermore, when IgA nephropathy is examined by the renal disease testing method of the invention, it is preferable that the detected amount of the complex used for determining an existence of IgA nephropathy is the ratio of the detected amount of the complex to the total amount of urinary proteins in the sample derived from urine collected from a subject. The ratio may be any value that is obtained by dividing the measured value of the detected amount of the complex by the measured value of the total amount of urinary proteins in the sample derived from urine collected from a subject, or the detected amount of the complex that is obtained as a relative value compared to the total amount of urinary proteins. By carrying out the determination based on this ratio, IgA nephropathy may be determined with higher sensitivity and specificity.
Furthermore, regarding the sample derived from urine collected from a subject from which the total amount of urinary proteins is measured, as long as it is obtained from the same subject, it may be a sample different from the sample derived from urine that is used for the detection of the complex as described above.

The determination step of the renal disease testing method of the invention preferably includes a step of comparing the detected amount of the complex of the antigen derived from human renal mesangial cells and human IgA in a sample derived from urine collected from a subject with the detected amount of the complex in samples derived from urine of healthy persons as a control, and a step of correlating a case in which the detected amount of the complex in the sample derived from urine collected from a subject is higher than the detected amount of the complex in the sample derived from urine of healthy persons as a control, with the existence of a renal disease.

Herein, the healthy persons as a control mean individuals who have previously been determined to have no occurrence of a renal disease. In addition; the expression "detected amount is higher" indicates that the detected amount of the complex derived from a subject is higher than the normal detected amount (i.e., a cut-off value) that is established in order to distinguish a healthy person from a patient having a renal disease.

The cut-off value may be determined by, for example, subjecting the detected amount of the complex in a sample group derived from urine of a patient having a renal disease and the detected amount of the complex in a sample group derived from urine of healthy persons as a control to ROC analysis, or the like. ROC analysis is an analytical method allowing, for example, the evaluation of detectability and diagnosability of a method of examining a disease. It is an analysis method described in Journal of The Japan Society for Clinical Laboratory Automation, "Manual for evaluation of diagnostic usefulness of clinical test" Ver.1.3 (2004.9.1), Vol. 29 Suppl. 1 (Serial Number 154) (published on Sep 1, 2004), for example.
Furthermore, the cut-off value may be defined as a value that is obtained by adding two or three times of the standard deviation to a mean detection level in healthy persons, or it may be suitably defined as a value which satisfies both the sensitivity (detection ratio) and specificity (i.e., low false positive ratio) in balance.

Examples of the renal disease in the invention include IgA nephropathy (IgAN), membranous nephropathy (MN), lupus nephropathy (SLE), focal glomerulosclerosis (FGS), minimal change nephritic syndrome (MCNS), diabetic nephropathy (DMN), amyloidosis, hereditary nephropathy (Alport), burnt-out IgA nephropathy (spontaneously remitted state of IgA nephropathy), membranoproliferative glomerulonephritis (MPGN), nephritis related with anti-neutrophil cytoplasmic antibody (ANCA), thin basement membrane disease (TBMD) and nephrosclerosis, etc.
According to the renal disease testing method of the invention, by detecting the complex in urine, various renal diseases including IgA nephropathy may be detected.

The IgA nephropathy testing method of the invention is **characterized in that** it includes a complex detection step of detecting a complex of an antigen derived from human renal mesangial cells and human IgA in a sample derived from urine collected from a subject, a first determination step of assessing the existence of a renal disease based on the amount of the complex detected in the complex detection step or the amount of urinary proteins in the sample derived from urine collected from a subject, and a second determination step of assessing whether the renal disease is IgA nephropathy based on the ratio of the amount of the complex detected in the complex detection step to the amount of urinary proteins in the sample derived from urine collected from a subject.
By assessing the existence of IgA nephropathy based on the ratio of the detected amount of the complex to the total amount of urinary proteins in the sample derived from urine, the existence of IgA nephropathy may be conveniently and safely determined with high sensitivity and high specificity, even at an early stage of the disease at which complete remission may be obtained by therapeutic intervention.

The complex detection step according to the invention is the same as the complex detection step for the renal disease testing method described above.
Furthermore, as for the method of detecting an amount of urinary proteins, if it allows the measurement of the total amount of urinary proteins in the sample derived from urine, a method for detecting proteins in a sample derived from urine that is generally performed in the art may be used without specific limitation. For example, the method described in the literature ("Revised edition of outlines for clinical test, 32nd edition" by Kanai Mitsumasa et al., pages 173-174, 2005) may be used.

With respect to the first determination step of the invention, determining the existence of a renal disease based on the amount of the complex detected in the complex detection step is the same as the determination step of the renal disease testing method described above.
Furthermore, the step of assessing the existence of a renal disease based on the amount of urinary proteins in the sample derived from urine preferably comprises a step of correlating the case in which the amount of urinary proteins in a sample derived from urine collected from a subject is higher than the normal detected amount (i.e., cut-off value) that is established in order to distinguish a patient having a renal disease from normal persons, with the existence of a renal disease.
Herein, the normal detected amount of urinary proteins may be defined by a general method.

The secondary determining step of the invention preferably comprises a step of comparing the ratio of the detected amount of the complex in a sample derived from urine collected from a subject to the total protein amount in the sample (i.e., the complex/total protein) with the ratio of the detected amount of the complex in a sample derived from urine collected from subjects who have a renal disease other than IgA nephropathy as a control to the total protein amount in the sample, and correlating the case in which the ratio of the detected amount of the complex in a sample derived from urine collected from a subject to the total protein amount in the sample is higher than the ratio of the detected amount of the complex in a sample derived from urine collected from subjects who have a renal disease other than IgA nephropathy as a control to the total protein amount in the sample, with the existence of IgA nephropathy.

Herein, the higher ratio means that the detected amount ratio of the complex derived from a subject is higher than the cut-off value that is determined to distinguish IgA nephropathy from renal diseases other than IgA nephropathy.
The cut-off value may be determined to have the same value as the cut-off value described in detail above for the renal disease testing method. However, from the viewpoint of detection specificity, the cut-off value is preferably determined based on the results of ROC analysis.

According to the IgA nephropathy testing method of the invention, the detected amount of the complex and total protein amount in urine are measured and IgA nephropathy may be assessed based on the ratio of the detected amount of the complex to the total protein amount. Therefore, it is a convenient and safe method of detecting IgA nephropathy.

The method of detecting the complex of an antigen derived from human renal mesangial cells and human IgA of the invention includes a step of bringing the sample derived from urine collected from a subject into contact with an antibody against an antigen derived from human renal mesangial cells and into contact with an antibody against human IgA. As a result, the complex of the antigen derived from human renal mesangial cells and human IgA may be detected with high sensitivity and high specificity.
Those described in detail above regarding the complex detection step for the renal disease testing method may be also applied to the method of detecting the complex of this invention.

Furthermore, the subject is preferably a person who has developed a renal disease, a person who is suspected to have developed a renal disease, or a person who has the possibility of developing a renal disease. Herein, a person who has the possibility of developing a renal disease means a person who is neither a person who has developed a renal disease nor a person who is suspected to have developed a renal disease. Still furthermore, the renal disease is more preferably IgA nephropathy.

The test kit for a renal disease of the invention is **characterized in that** it comprises at least one antibody against an antigen derived from human renal mesangial cells and at least one antibody against human IgA. By detecting the complex of an antigen derived from human renal mesangial cells and human IgA using the antibody against the antigen derived from human renal mesangial cells and the antibody against human IgA, the renal disease may be detected with good detection sensitivity and good specificity.
It is preferable that the test kit for a renal disease of the invention further comprises an instruction which describes brining the sample derived from urine collected from a subject into contact with the antibody against the antigen derived from human renal mesangial cells and the antibody against human IgA to detect the complex of the antigen derived from human renal mesangial cells and human IgA, and correlating the detected amount with an existence of a renal disease.
Furthermore, the test kit for a renal disease of the invention may be preferably used for assessing an existence of IgA nephropathy.

Disclosure of the Japanese Patent Application No. 2008-144883 is incorporated by reference herein in its entirety.
All publications, patent applications, and technical standards that are described in the specification are herein incorporated by reference to the same extent as if each individual publication, patent application, or technical standard was specifically and individually indicated to be incorporated by reference.

### EXAMPLES

In the following, the present invention is explained specifically with reference to examples. However, the invention is not limited by the examples. Furthermore, unless specifically mentioned otherwise, "%" represents mass percentage.

### [Example 1]

### <Obtainment of a monoclonal antibody that recognizes a human renal mesangial cell antigen>

Normal human renal mesangial cells (trade name: Cryo NHMC, manufactured by Cambrex Corporation) were cultured. A BALB/c mouse (female) was immunized with the cultured cells or the fraction extracted from the cytoskeletal component. After confirming the increase in the titer against the immunogen, spleen cells were subjected to cell fusion with P3U-1 cells to prepare a hybridoma. The first screening of the hybridoma was performed by EIA using two kinds of immunogenic antigen (normal human renal mesangial cells or the fraction extracted from the cytoskeletal component) immobilized on cups of micro plates. Then, hybridomas which exhibited high values of color development for one or both of the antigens were selected. Meanwhile, the fraction extracted from the cytoskeletal component was prepared using the ProteoExtract Subcellular Proteome Extraction Kit (trade name, manufactured by CalBiochem).
The selected hybridomas were prepared as monoclones based on a limiting dilution method. Thereafter, to select hybridomas that are specific to human renal mesangial cells, the second screening was performed using the monoclones. The second screening of the hybridomas was carried out by ELISA such that the clones having a response to the immobilized normal human renal mesangial cells or the fraction extracted from the cytoskeletal component but not to the epithelial cells of the human proximal renal tubule (trade name: Cryo RPTEC, manufactured by Calbiochem), which were cultured as a control, or the immobilized fraction extracted from the cytoskeletal component of the epithelial cells were selected.

ELISA was performed as follows. Each of the normal human renal mesangial cells and the normal epithelial cells of the human proximal renal tubule were added (4,000 cells/200 µL) to PolySorp cups (manufactured by NUNC, Inc.) and then cultured overnight at 37°C. The cup was washed five times with PBS (pH 7.2), 0.05% glutaraldehyde solution was added (100 µL/well), and reacted at room temperature for 30 minutes. The cups were washed five times with a washing solution (50 mM Tris/HCl (pH 7.5), 150 mM NaCl and 0.01% Tween20) and then a blocking solution was added (10% normal rabbit blood serum (manufactured by Pel-Freeze Biologicals), 50 mM Tris/HCl (pH 7.5), 0.15 M NaCl, 10 mM-EDTA-2Na, 0.01 % Tween20 and 0.1% NaN₃) in an amount of 150 µL/well. Thereafter, the blocking was carried out at room temperature for 2 hours or at 4°C for one day or more (in the following, referred to as "cups for antibody screening").
Meanwhile, the fraction extracted from the normal human renal mesangial cells and the fraction extracted from the skeletal component of the normal epithelial cells of the human renal proximal tubule were each diluted to the concentration of 10 µg/mL using 50 mM Tris/HCl (pH 7.5) and 0.15 M NaCl, and then added to PolySorp cups (manufactured by NUNC, Inc.) (50 µL/well). The cups were placed in a humid box and coating was carried out overnight at 4°C. Processes after the coating were carried out in the same manner as the cups for antibody screening as described above.

Each of four kinds of the cups for antibody screening was washed three times with the washing solution (50 mM Tris/HCl (pH 7.5), 150 mM NaCl and 0.01% Tween20), added with hybridoma culture supernatant (50 µL/well), and the reaction was carried out for 1 hour at room temperature. After washing three times with the washing solution (50 mM Tris/HCl (pH 7.5), 150 mM NaCl and 0.01% Tween20), two kinds of labeled antibody, i.e., HRP-Rabbit Anti-Mouse IgG (trade name, manufactured by Zymed Labs, diluted 1,000 times) and HRP-Rabbit Anti-Mouse IgG1 (trade name, manufactured by Zymed Labs, diluted 4,000 times), each diluted in 25% normal rabbit blood serum (manufactured by Pel-Freeze Biologicals) buffered with 0.2 M Na₂HPO₄/0.1M citrate (pH 5.4), were added in an amount of 50 µL/well followed by reaction at room temperature for 1 hour. After washing three times with the washing solution, 3,3',5,5'-Tetramethylbenzidine (TMB) Liquid Substrate System for ELISA (trade name, manufactured by Sigma Chemical Corporation) was added in an amount of 100 µL/well followed by reaction at room temperature for 30 minutes. After adding 0.5 M H₂SO₄ (100 µL/well) to terminate the reaction, OD (450 to 650 nm) was measured. Regarding the results of this measurement, in particular when HRP-Anti-Mouse IgGl was used, the clones showing the high values of color development for both or any one of the normal human renal mesangial cells and the fraction extracted from the cytoskeletal component but the low values of color development for both of the normal epithelial cells of the human renal proximal tubule and the fraction extracted from the cytoskeletal component were selected.

### <Determination of the reaction characteristics of the monoclonal antibody>

The hybridoma cells obtained from the above were cultured. From the ascites obtained from transplantation of the cells to a mouse abdominal cavity, IgG was purified by using PROSEP-A (trade name, manufactured by Millipore Corp.). Specificity of the purified monoclonal antibody was determined by the method which is the same as the ELISA used for the screening above.

In an immobilized state, five kinds of purified monoclonal antibody (4H3-1D12-1D2, 4H3-2E7-1D2, 6C4-6A8-1B4, 14E11-2G7-1B2 and 11A11-2F9-1G3) showed high values of color development for the normal human renal mesangial cells and the fraction extracted from the cytoskeletal component, but showed low values of color development for normal epithelial cells of the human renal proximal tubule (trade name: Cryo NHMC, manufactured by Cambrex Corporation) as a control and the immobilized fraction of the extract from the cytoskeletal component.

Next, the fraction extracted from the cytoskeletal component of normal human renal mesangial cells was subjected to SDS-PAGE and blotted on a nitrocellulose filter (trade name: BA85, manufactured by Schleicher & Schuell BioScience, GMBH.). The filter was immersed in a blocking solution (50 mM Tris-HCl (pH 7.5), 150 mM NaCl, 5% skim milk, 0.01% Tween20 and 0.1% NaN₃) and shaken overnight at 4°C. After exchanging the blocking solution, culture supernatant of each clone of the monoclonal antibody obtained above was added and shaken at room temperature for 2 hours followed by washing three times with the washing solution. To the washing solution, HRP-labeled anti-mouse IgG (manufactured by Zymed Laboratories, Inc.) of a secondary antibody was added in 1/1000 concentration. After shaking at room temperature for 1 hour and washing three times with the washing solution, a solution for color development (8.3mM Tris-HCl (pH 6.5), 125 mM NaCl, 0.05% 4-chloro-1-naphtol and 0.01% H₂O₂) was added thereto for color development.

As a result of the above Western blot analysis, it was found that each of the 4H3 and 6C4 clones reacted with components having different molecular weights from the fraction extracted from human renal mesangial cells. It is believed that each of the 4H3 and 6C4 clones recognize different antigens derived from human renal mesangial cells.

### <Detection of renal disease>

### Detection of IgA-6C4 recognizing antigen complex based on ELISA

6C4-6A8-1B4 antibody (in the following, referred to as "6C4") was purified by PROSEP-A (trade name, manufactured by Millipore Corp.) followed by dialysis against 50 mM Tris/HCl (pH 7.5) and 0.15 M NaCl. The purified antibody was diluted to the concentration of 5 to 10 µg/mL by using 50 mM Tris/HCl (pH 7.5) and 0.15 M NaCl, and then added to a PolySorp cups (manufactured by NUNC, Inc.) by 50 µL/well. The cups were placed in a humid box and coating was carried out overnight at 4°C. After the coating, they were washed three times with the washing solution (50 mM Tris/HCl (pH 7.5), 150 mM NaCl and 0.01% Tween20) followed by addition of the blocking solution (50% N102 (trade name, manufactured by NOF Corp.), 25 mM Tris/HCl (pH 7.5), 75 mM NaCl and 2% Block Ace (trade name, manufactured by Dainippon Sumitomo Pharma Co., Ltd.)) by 150 µL/well. Thereafter, the blocking was carried out at room temperature for 2 hours or at 4°C for one day or more (herein below, referred to as "6C4-coated cups").

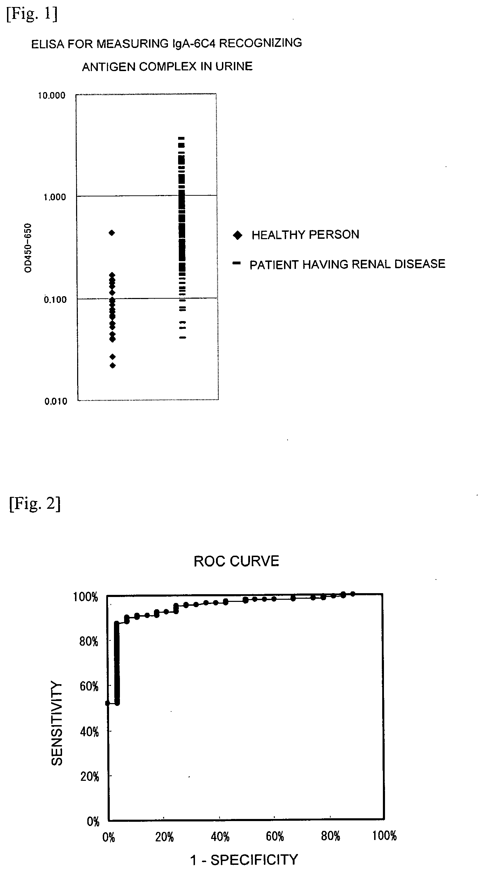

6C4-coated cups were washed three times with the washing solution (50 mM Tris/HCl (pH 7.5), 150 mM NaCl and 0.01% Tween20) and added with urine samples obtained by 50 times dilution using a sample dilution solution (50% N102 (trade name, manufactured by NOF Corp.), 50 mM Tris/HCl (pH 7.5), 150 mM NaCl and 2% Block Ace (trade name, manufactured by Dainippon Sumitomo Pharma Co., Ltd.) in an amount of 50 µL/well for the reaction at room temperature for 1 hour. After washing three times with the washing solution (50 mM Tris/HCl (pH 7.5), 150 mM NaCl and 0.01 % Tween20), HRP-GOAT Anti-Human IgA (trade name, manufactured by Zymed Labs) which was diluted 3,000 times with Can Get Signa12 (trade name, manufactured by Toyobo Inc.) was added in an amount of 50 µL/well, and the reaction was carried out at room temperature for 1 hour. After washing three times with the washing solution, 3,3',5,5'-Tetramethylbenzidine (TMB) Liquid Substrate System for ELISA (trade name, manufactured by Sigma Chemical Corporation) was added in an amount of 100 µL/well followed by reaction at room temperature for 30 minutes. After adding 0.5 M H₂SO₄ (100 µL/well) to terminate the reaction, OD (450 to 650 nm) was measured. Measurement results obtained from 146 cases of patients having renal diseases including 95 cases of patients having IgA nephropathy and 28 cases of healthy persons (normal persons) are shown in Fig. 1.

Comparing the value obtained after subtraction of blank in Fig. 1, results showing a significant difference between 146 cases of patients having a renal disease including 95 cases of patients having IgA nephropathy and 28 cases of healthy persons (normal persons) were obtained.
Furthermore, as a result of carrying out ROC analysis for 146 cases of patients having a renal disease and 28 cases of healthy persons, the ROC curve shown in Fig. 2 was obtained. The cut-off value calculated from the ROC curve was 0.155. The positive response ratio in 146 cases of patients having a renal disease and 28 cases of healthy persons, which was determined from the cut-off value, is shown in Table 1. As shown in Table 1, there were 132 cases showing a positive response (90.4%) out of 146 cases of patients having a renal disease while 2 cases showing a positive response (7.1 %) out of 28 cases of healthy persons, thus two groups can be clearly distinguished from each other. The sensitivity was 90.4%, specificity degree (specificity) was 92.9%, and diagnosis efficiency was 90.8%.

**Table 1**

| | Patient having a renal disease | Healthy person |
|---|---|---|
| Number of samples | 146 | 28 |
| Number of positive response | 132 | 2 |
| Positive response ratio | 90.40% | 7.10% |

### <Detection of IgA nephropathy>

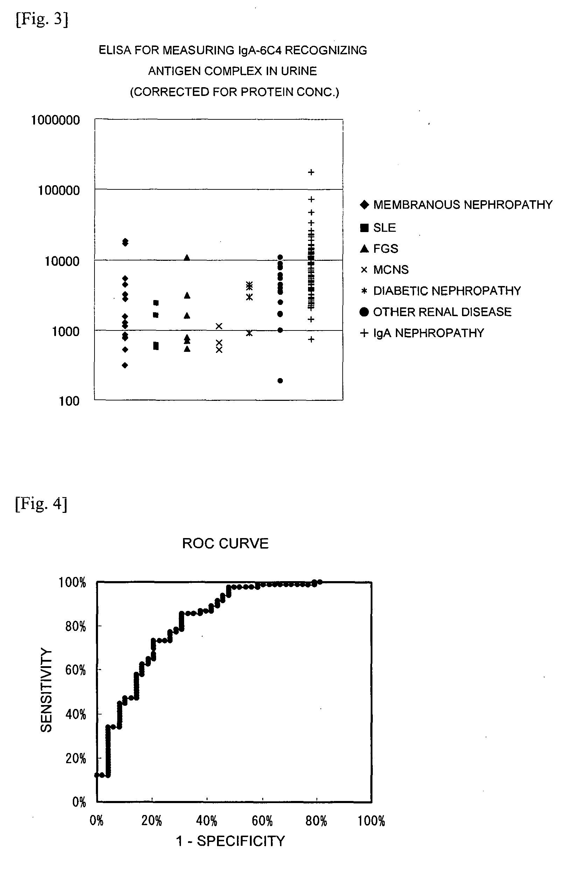

Next, for the samples in which the detected amount of the complex exhibited color development values higher than the cut-off value above, protein concentration in urine was quantified based on a pyrogallol red method. The detected amount of IgA-6C4 recognizing antigen complex (i.e., the value shown in Fig. 1), which has been detected from the above, was divided by the protein concentration, and then the detected amount of the complex per protein concentration in urine was calculated and shown in Fig. 3. It was found from Fig. 3 that 83 cases of patients having IgA nephropathy can be clearly distinguished from 48 cases of patients having a renal disease other than IgA nephropathy.
Furthermore, as a result of carrying out ROC analysis for 83 cases of patients having IgA nephropathy and 48 cases of patients having a renal disease other than IgA nephropathy, the ROC curve shown in Fig. 4 was obtained. The cut-off value calculated from the ROC curve was 4,509. The positive response ratio in 83 cases of patients having IgA nephropathy and 48 cases of patients having a renal disease other than IgA nephropathy, which was determined from the cut-off value, is shown in Table 2. As shown in Table 2, there were 61 cases showing a positive response (73.5%) out of 83 cases of patients having IgA nephropathy while 10 cases showing a positive response (20.8%) out of 48 cases of patients having a renal disease other than IgA nephropathy, thus two groups can be clearly distinguished from each other. The sensitivity was 73.5%, specificity degree was 79.2%, and diagnosis efficiency was 75.6%.

**Table 2**

| | IgA nephropathy | Renal disease other than IgA nephropathy |
|---|---|---|
| Number of samples | 83 | 48 |
| Number of positive response | 61 | 10 |
| Positive response ratio | 73.50% | 20.80% |

### [Example 2]

### <Detection of renal disease>

### - Detection of IgA-4H3-recognizing antigen complex based on ELISA -

4H3-1D12-1D2 antibody (herein below, referred to as "4H3") was purified by PROSEP-A (trade name, manufactured by Millipore Corp.) followed by dialysis against 50 mM Tris/HCl (pH 7.5) and 0.15 M NaCl. The purified antibody was diluted to the concentration of 5 to 10 µg/mL by using 50 mM Tris/HCl (pH 7.5) and 0.15 M NaCl, and then added to a PolySorp cups (manufactured by NUNC, Inc.) by 50 µL/well. The cups were placed in a humid box and coating was carried out overnight at 4°C. After coating, they were washed three times with a washing solution (50 mM Tris/HCl (pH 7.5), 150 mM NaCl and 0.01% Tween20) followed by addition of a blocking solution (50% N102 (trade name, manufactured by NOF Corp.), 25 mM Tris/HCl (pH 7.5), 75 mM NaCl and 2% Block Ace (trade name, manufactured by Dainippon Sumitomo Pharma Co., Ltd.)) by 150 µL/well. Thereafter, blocking was carried out at room temperature for 2 hours or at 4°C for one day or more (herein below, referred to as a "4H3-coated cups").

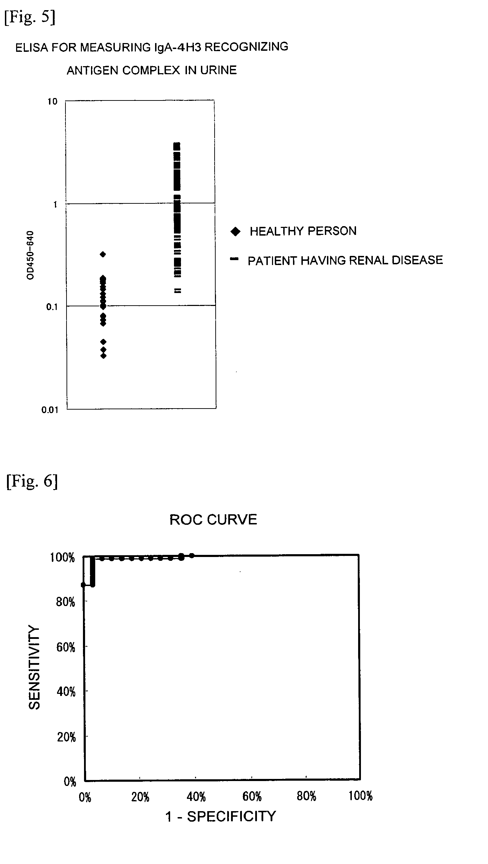

4H3-coated cups were washed three times with washing solution (50 mM Tris/HCl (pH 7.5), 150 mM NaCl and 0.01% Tween20) and added with urine samples which were obtained by 50 times dilution using a sample dilution solution (50% N102 (trade name, manufactured by NOF Corp.), 50 mM Tris/HCl (pH 7.5), 150 mM NaCl and 2% Block Ace (trade name, manufactured by Dainippon Sumitomo Pharma Co., Ltd.)) in an amount of 50 µL/well for a reaction at room temperature for 1 hour. After washing three times with washing solution (50 mM Tris/HCl (pH 7.5), 150 mM NaCl and 0.01% Tween20), HRP-GOAT Anti-Human IgA (trade name, manufactured by Zymed Labs) which has been diluted 3,000 times with Can Get Signa12 (trade name, manufactured by Toyobo Inc.) was added in an amount of 50 µL/well, and the reaction was carried out at room temperature for 1 hour. After washing three times with the washing solution, 3,3',5,5'-Tetramethylbenzidine (TMB) Liquid Substrate System for ELISA (trade name, manufactured by Sigma Chemical Corporation) was added in an amount of 100 µL/well followed by reaction at room temperature for 30 minutes. After adding 0.5 M H₂SO₄ (100 µL/well) to terminate the reaction, OD (450-650 nm) was measured. Measurement results obtained from 149 cases of patients having a renal disease, including 95 cases of patients having IgA nephropathy, and 28 cases of healthy persons are shown in Fig. 5.

Comparing the value obtained after subtraction of blank in Fig. 5, results showing a significant difference between 149 cases of patients having a renal disease, including 95 cases of patients having IgA nephropathy, and 28 cases of healthy persons were obtained.
Furthermore, as result of carrying out ROC analysis for 149 cases of patients having a renal disease and 28 cases of healthy persons, the ROC curve shown in Fig. 6 was obtained. The cut-off value calculated from the ROC analysis was 0.192. The positive response ratio in 149 cases of patients having a renal disease and 28 cases of healthy persons, which was determined from the cut-off value, is shown in Table 3. As shown in Table 3, there were 147 cases showing a positive response (98.7%) out of 149 cases of patients having a renal disease while 1 case showing a positive response (3.6%) out of 28 cases of healthy persons, thus two groups can be clearly distinguished from each other. The sensitivity was 98.7%, specificity degree was 96.4%, and diagnosis efficiency was 98.3%.

**Table 3**

| | Patient having a renal disease | Healthy person |
|---|---|---|
| Number of samples | 149 | 26 |
| Number of positive response | 147 | 1 |
| Positive response ratio | 98.70% | 3.60% |

### <Detection of IgA nephropathy>

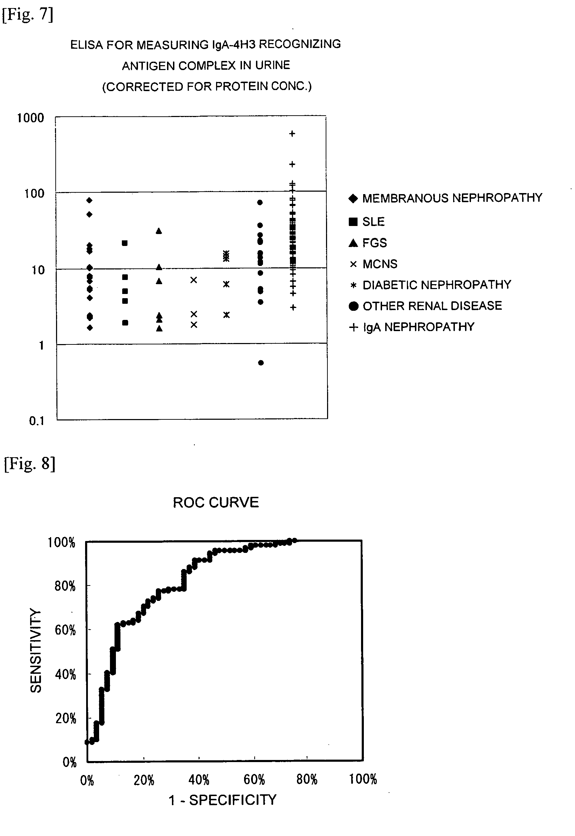

Next, for the samples in which the color development values were higher than the cut-off value above, protein concentration in urine was quantified based on a pyrogallol red method. The detected amount of IgA-4H3 recognizing antigen complex (i.e., the value shown in Fig. 5), which was detected from the above, was divided by the protein concentration, and then the detected amount of the complex per protein concentration in urine was calculated and shown in Fig. 7. It was found from Fig. 7 that 92 cases of patients having IgA nephropathy can be clearly distinguished from 54 cases of patients having a renal disease other than IgA nephropathy.
Furthermore, as result of carrying out ROC analysis for 92 cases of patients having IgA nephropathy and 54 cases of patients having a renal disease other than IgA nephropathy, the ROC curve shown in Fig. 8 was obtained. The cut-off value calculated from the ROC curve was 15.07. The positive response ratio in 92 cases of patients having IgA nephropathy and 54 cases of patients having a renal disease other than IgA nephropathy, which was determined from the cut-off value, is shown in Table 4. As shown in Table 4, there were 71 cases showing a positive response (77.2%) out of 92 cases of patients having IgA nephropathy while 14 cases showing a positive response (25.9%) out of 48 cases of patients having a renal disease other than IgA nephropathy, thus two groups can be clearly distinguished from each other. The sensitivity was 77.2%, specificity degree was 74.1 %, and diagnosis efficiency was 76.0%.

**Table 4**

| | IgA nephropathy | Renal disease other than IgA nephropathy |
|---|---|---|
| Number of samples | 92 | 54 |
| Number of positive response | 71 | 14 |
| Positive response ratio | 77.20% | 25.90% |

From the above, it was found that, by detecting the complex of an antigen derived from human renal mesangial cells and human IgA present in urine, healthy persons and patients having a renal disease may be differentiated from each other.
Furthermore, it was also found that, by measuring the detected amount of the complex of an antigen derived from human renal mesangial cells and human IgA present in urine per urinary protein concentration, IgA nephropathy may be differentiated from other renal diseases.

## Claims

1. An IgA nephropathy testing method, comprising:
a step of detecting a complex of an antigen derived from human renal mesangial cells and human IgA in a sample derived from urine collected from a subject.

2. The testing method according to claim 1, wherein the detecting of the complex comprises bringing the sample into contact with an antibody against the antigen derived from human renal mesangial cells and an antibody against human IgA.

3. The testing method according to claim 1 or 2, further comprising:
a step of obtaining a ratio of an amount of the complex detected in the complex detection step to an amount of urinary proteins in the sample derived from urine collected from the subject.

4. The testing method according to claim 3, further comprising:
a step of assessing the existence of IgA nephropathy on the basis of the ratio.

5. A renal disease testing method, comprising:
a step of detecting a complex of an antigen derived from human renal mesangial cells and human IgA in a sample derived from urine collected from a subject.

6. The testing method according to claim 5, wherein the complex detection step comprises bringing the sample into contact with an antibody against the antigen derived from human renal mesangial cells and an antibody against human IgA.

7. The testing method according to claim 5 or 6, further comprising:
a step of assessing the existence of a renal disease on the basis of the detected amount of the complex.

8. An IgA nephropathy testing method, comprising:
a step of detecting a complex of an antigen derived from human renal mesangial cells and human IgA in a sample derived from urine collected from a subject;
a step of assessing the existence of a renal disease on the basis of the amount of the complex detected in the complex detection step or an amount of urinary proteins in the sample; and
a step of assessing whether the renal disease is IgA nephropathy on the basis of a ratio of the amount of the complex detected in the complex detection step to the amount of urinary proteins in the sample.

9. The testing method according to any one of claims 1 to 8, wherein the complex detection step comprises an immunochemical method using an antibody against an antigen derived from human renal mesangial cells and an antibody against human IgA.

10. The testing method according to claim 9, wherein the immunochemical method is a sandwich method.

11. A method of detecting a complex of an antigen derived from human renal mesangial cells and human IgA, the method comprising:
a step of bringing a sample derived from urine collected from a subject into contact with an antibody against an antigen derived from human renal mesangial cells and an antibody against human IgA.

12. The detection method according to claim 11, wherein the complex is detected by an immunochemical method using the antibody against the antigen derived from human renal mesangial cells and the antibody against human IgA.

13. The detection method according to claim 12, wherein the immunochemical method is a sandwich method.

14. The detection method according to any one of claims 11 to 13, wherein the subject is a person who has developed a renal disease, a person who is suspected to have developed a renal disease, or a person who has a possibility of developing a renal disease.

15. The detection method according to claim 14, wherein the renal disease is IgA nephropathy.

16. A test kit for a renal disease, comprising:
an antibody against an antigen derived from human renal mesangial cells; and
an antibody against human IgA.

17. The test kit according to claim 16, wherein the renal disease is IgA nephropathy.
